# EUROPEAN PATENT SPECIFICATION

(11) **EP 2 528 003 B1**
(45) Date of publication and mention of the grant of the patent: **07.08.2019**
(21) Application number: 12158977.4
(22) Date of filing: 12.03.2012
(51) Int. Cl.: A61M 5/00, A61M 5/142, A61M 5/145

(54) **Architecture and method for controlling and programming an electromedical apparatus**
Architektur und Verfahren zur Steuerung und Programmierung einer elektromedizinischen Vorrichtung
Architecture et procédé de commande et de programmation d'un appareil électromédical

(30) Priority: 11.03.2011 IT TO20110223
(43) Date of publication of application: 28.11.2012
(73) Proprietor: Cane' S.p.A., 10098 Rivoli (TO) (IT)
(72) Inventor: Cane', Mario, 10095 COLLEGNO (TO) (IT)
(74) Representative: Robba, Pierpaolo

(56) References cited:
- WO-A1-99/62403
- WO-A1-03/019185
- WO-A2-03/038566
- WO-A2-2004/024214
- WO-A2-2004/034886
- WO-A2-2005/025646

## Description

### Filed of the art

The present invention concerns an architecture and a method for controlling and programming an electromedical apparatus.

More precisely, the invention concerns an architecture and a method for controlling and programming the mode of operation of an electromedical apparatus, of the type which can be programmed to operate in accordance with a predetermined therapy.

### Prior art

Generally speaking an electromedical apparatus consists of an electronic or electromechanical device, which interacts with the patient's body and which is capable of operating in accordance with a predetermined therapy.

Electromedical apparatus are currently used on both human beings and animals.

The invention refers to programmable electromedical apparatus comprising e.g. electromechanical devices for drug infusion. Drug infusion devices are disclosed e.g. in IT-A-1261234 and IT-A-1257247. Other examples of electromedical devices with which the invention can be advantageously employed are electrostimulators. The invention can be further employed in association with substantially any kind of electromedical device capable of being programmed to operate in accordance with a predetermined therapy.

Infusion devices, also known as drug infusion pumps, are employed in the medical sector for administering liquid drugs to a patient.

These electromedical devices generally provide for the drug contained in a reservoir, for example a syringe filled when necessary or a prefilled ampoule, to be administered to the patient through a device, for example a needle or a cannula, pierced into the patient's body itself.

It is also known that these electromedical devices are programmed to operate in accordance with a predetermined therapy. For example, these devices are programmed for administering the drug during a predetermined period of time, e.g. twenty-four hours, with flows which vary during the day, so as to cope with the change of conditions of the patient and with the physical activities performed. These devices are generally programmed by specialized staff, typically medical staff.

One of the problems encountered during the use of electromedical devices in general and in particular of drug infusion pumps, arises from the fact that in many cases it would be desirable that the therapy were programmed according to the actual health conditions of the patient. Such conditions are variable in time because of a number of factors. For example, the effectiveness of the therapy itself may require the program to be modified, or the health conditions of the patient or the different physical activity performed by the patient may cause the need to vary the programmed therapy.

By contrast, the administration therapy is generally programmed on the basis of preexisting clinical parameters, e.g. the results of various analysis and tests, with reference to theoretical expectations calculated on the basis of experience and literature in the field, often without considering, or considering only a small part of, the actual health conditions of the patient and the variations of his/her status, as long as the therapy is carried out.

Currently it is also known to collect data on several relevant parameters associated with the health status of the patient by means of record cards, which are manually filled in by the patient himself or by an assistant. These data are then analyzed by medical staff and sometimes used to modify the profile of the therapy administered through the infusion device.

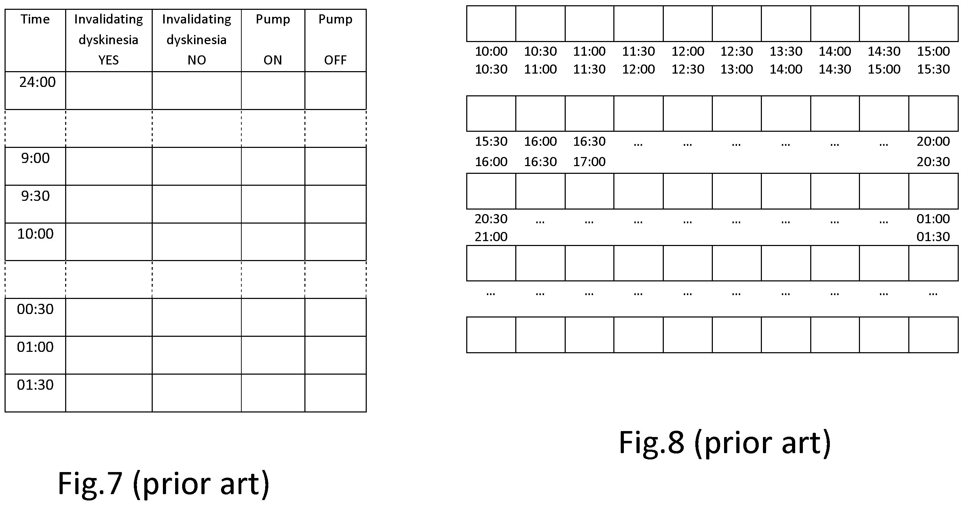

In case of patients suffering from Parkinson disease it is known to collect data on record cards where for each time interval (e.g. 30 min.) one has to report if the mobility capacity of the patient is good or not, if rigidity conditions are absent or not, if an invalidating dyskinesia is present, etc... These particular conditions of the patient can be reported by ticking a specific box on the record card, or by making use of a symbol indicative of the condition which has to be reported (e.g. "d" for moderate dyskinesia, "D" for serious dyskinesia, etc...). Figs 7 and 8 show examples of record cards used in the first and second case, respectively.

It is evident that data collection by means of record cards does not allow a real time analysis and delays the time needed to modify the therapy. Furthermore, this type of data collection does not allow the physician to quickly appreciate the correlation between the patient's conditions, reported on the filled record card, and the corresponding flow regime programmed in the pump within the same time span. To solve the above problem, it has been proposed in the past to display the profile of the therapy in various graphical patterns. These graphic visualizations associated with information acquired on the variation of the health status of the patient, can be used by the physician to intervene on the device program by changing its mode of operation.

US 2010/0265072 A1 discloses techniques for displaying representations of administration sessions of an electromedical device, in particular a drug infusion device. Information regarding the administration sessions may be stored in the infusion device and downloaded by a programmer or a physician in order to be displayed to a user. For example, a programmer can download these data from the memory of the device through a wireless communication and display them so that they can be analyzed. By exploiting its own knowledge and the information received from the device, the medical staff can decide whether and how to modify the administration therapy of the drug to the patient.

WO 03/038566 discloses a user interface for the delivery of sedation and analgesia wherein the use of a hybrid display is proposed having both the function of a touch-screen and buttons provided with keys for the most critical functions. Both historical and real-time information on some parameters pertaining to the patient (e.g. hemodynamic / cardiovascular, oxigenation, breath ventilation) are displayed on the screen. With the aim of facilitating the understanding by the physician, this information is displayed on the screen in parallel rows containing homogenous data.

WO 2004/024214 A2 discloses a drug delivery system comprising a display for showing the drug delivery and patient statistics and provided with several interfaces to control bolus delivery.

Other examples of modes of displaying the parameters measured on the patient and/or pertaining to the administered therapy are disclosed by WO 2005/025646, WO 03/019185, WO 99/62403 and WO 2004/034886.

One of the drawbacks of the known systems for managing the programming of electromedical devices arises from the circumstance that the physician, since no rapid instrument of analysis is available to him/her, is generally led to operate empirically by trying to modify the therapy by means of successive steps in the attempt to eventually obtain the best results.

Clearly, one of the main drawbacks of this mode of operating is caused by the fact that the therapy is not programmed in real time, i.e. according to the actual conditions of the patient, and the determination of the optimal therapy is obtained by successive approximations, which may require even a long time according to the availability of the medical staff.

By contrast, one of the main objects of the present invention is that of providing a solution to the above problem by supplying to the physician or in general to the programmer an architecture and a method for controlling and programming an electromedical device, enabling fast and effective operations.

E.g. in case of patients suffering from Parkinson disease the drawbacks of the known systems are particularly noticeable. It is known that some of the therapies used for patients suffering from Parkinson disease require the prolonged infusion of a drug by means of an infusion pump. The drug has to be administered to the patient substantially during the whole 24-hour period, often with different flows depending on the time slot so as to take into account the physical activity, e.g. the sleep or awake status, of the patient and preferably of any other factor which might influence the efficacy of the therapy.

Another object of the invention is therefore that of providing an architecture and a method for controlling and programming a drug infusion pump, that enable to improve the performances of drug administration therapies to patients suffering from Parkinson disease.

A further object of the invention is that of providing an architecture and a method of the above type, which turns to be safe and reliable for the patient.

The last but not least object of the invention is that of providing an architecture and a method of the above type, which can be industrially implemented at low costs.

### Disclosure of the invention

Advantageously the invention provides the medical staff, or more in general the programmer, with a fast and efficient tool which enables the optimal definition of the therapy parameters for controlling an electromedical device, in particular for administering a drug and more particularly, but not exclusively, apomorphine to patients suffering from Parkinson disease.

Advantageously, thanks to the method of the invention the therapy for administering and dosing the drug can be easily customized according to the actual needs of the patient, the lifestyle, the health conditions and the progress or regress of the disease.

Advantageously, the invention can be implemented in a mobile communication device, e.g. a handheld device equipped with a touch-screen.

Advantageously, according to a particular embodiment of the invention, the patient can report to the physician the variations of his/her health conditions in real time, e.g. at the same time he/she experiences these variations, thereby allowing the physician to be timely informed on the effects and efficacy of the therapy and to equally timely amend the therapy if necessary.

A further advantage of the invention derives from the possibility for the physician to remotely re-program the therapy, thus avoiding that the patient goes to the doctor or that the physician visits the patient.

Advantageously, thanks to the invention, the physician or the programmer will be able to store in an archive the data pertaining to the therapy, the signals and commands imparted offhand and the modifications imparted to the therapy, in case mutually comparing the data of the various patients. For example it will be possible to exploit the dosage used for a patient which provided encouraging results for programming the infusion unit of a second patient with similar health conditions.

A further advantage of the invention derives from the possibility to choose among a plurality of therapies, three according to a preferred embodiment of the invention, and/or to administer an additional dose of drug when necessary.

Advantageously, it is provided that the infusion unit and the display unit communicate among them in encrypted mode by means of proprietary protocols, thereby frustrating any attempt to communicate by other devices.

### Brief description of the drawings

Several preferred embodiments of the invention will be described hereinafter by way of example with reference to the accompanying drawings, in which:
- Fig.1 is a block diagram of the architecture according to the invention;
- Fig.2 shows a first screen of the signaling and control interface;
- Fig.3 shows a second screen of the signaling and control interface;
- Fig.4 shows a screen of the signaling and control interface in accordance with a first therapy;
- Fig.5 shows a second screen of the signaling and control interface in accordance with a second therapy;
- Fig.6 shows a third screen of the signaling and control interface in accordance with a third therapy;
- Figg. 7 and 8 are respective examples of record cards for data collection used in the prior art.

### Detailed description of a preferred embodiment

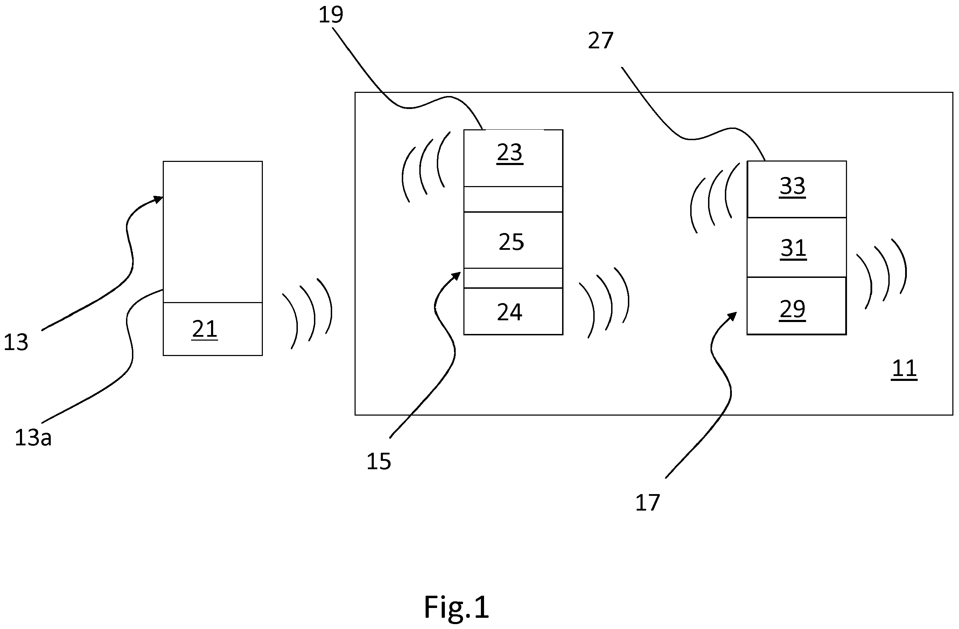

With reference to Fig.1 the architecture for controlling and programming according to the invention has been generally denoted with reference numeral 11.

Architecture 11 will be described in the following with reference to controlling and programming a drug infusion unit 13 comprising a drug infusion pump 13a; however, the invention can be employed substantially in any type of programmable electromedical device.

According to a preferred embodiment of the invention, said architecture 11 comprises a signaling and control unit 15 and a displaying and programming unit 17.

In the illustrated example, the signaling and control unit 15 is associated with a mobile apparatus 19 such as for example a wireless communication apparatus of the handheld type, i.e. having a size that fits in a hand palm.

Still referring to the illustrated example the infusion unit 13 and the mobile apparatus 19 are equipped with respective communication modules, 21 and 23 respectively, preferably of the wireless type, e.g. of the type which operates according to the Bluetooth technology, for short-range communication.

Furthermore, said signaling and control unit 15 will be equipped with a further wireless communication module 24, e.g. operating according to the GSM protocol, for long-range communication.

Furthermore, still with reference to the illustrated embodiment, preferably the signaling and control unit 15 comprises a touch-screen 25, i.e. a screen of the type obtained by means of the combination of a display and a digitalizer, which allows the user to interact with the graphic interface by means of his fingers.

Moreover, the displaying and programming unit 17 comprises a personal computer 27, e.g. of the portable type, equipped with a screen 31, a first wireless communication module 29, e.g. comprising a Bluetooth interface for short-range communication and/or a second wireless communication module 33, e.g. comprising a GSM interface, for long-range communication.

According to the invention the user of the infusion unit 13, typically the patient himself or in case a caregiver, e.g. a nurse, will use the signaling and control unit 15 for signaling variations of the conditions of the patient, e.g. indispositions provoked by blocks of functionality, tremor, or others, or more simply variations in the performed activity, e.g. wake, work, relax, motor activity. Through the signaling and control unit 15 it will be possible to signal the type, intensity and duration of phenomena, e.g. by sending several times the same signal in a short time span.

Still according to the invention, advantageously, the signaling and control unit 15 can be used to receive data on the working conditions of said unit 13 from the infusion unit 13 and for communicating with said unit, preferably by means of modules 23 and 24, for displaying through screen 25 the therapy in use, the residual duration of the infusion or the residual level of drug in the reservoir associated with the pump 13a, e.g. a syringe.

Still according to a preferred embodiment of the invention, advantageously the signaling and control unit 15 will allow controlling the infusion unit 13, e.g. for administering additional doses of drug (bolus), if necessary, thereby avoiding the need for a direct intervention on the infusion unit 13.

Advantageously, signaling and control unit 15 and displaying and programming unit 17 can communicate with each other through the respective modules 23 and 24, e.g. with a GSM protocol, in an encrypted mode, by exploiting the communication network provided for the mobile telephony.

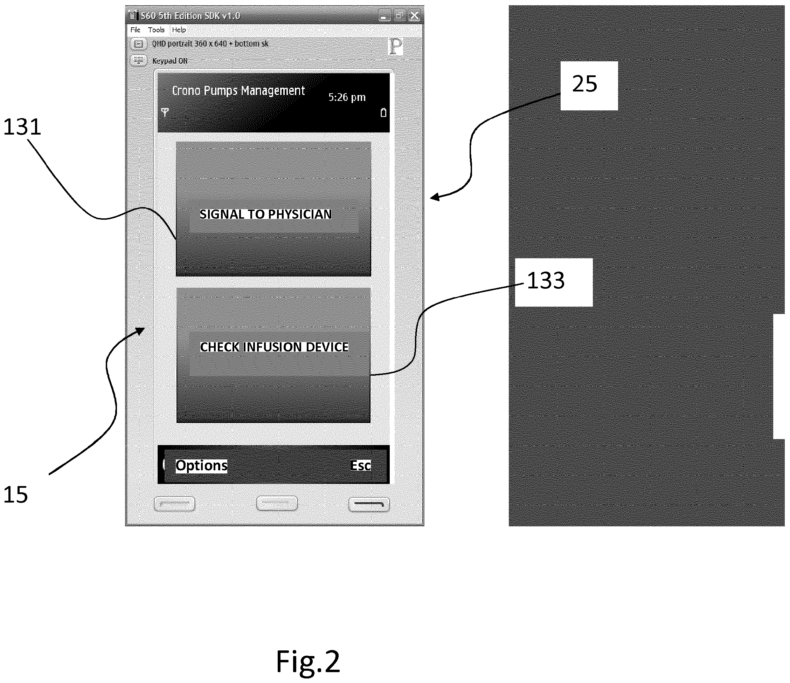

Referring to Fig.2 there is illustrated a first screen of the signaling and control interface associated with the signaling and control unit 15.

Said screen shows, in a touch-screen environment, the location of input command 131 to get access to the functions for sending a report to the physician and the location of input command 133 to get access to the functions for controlling the infusion unit 13.

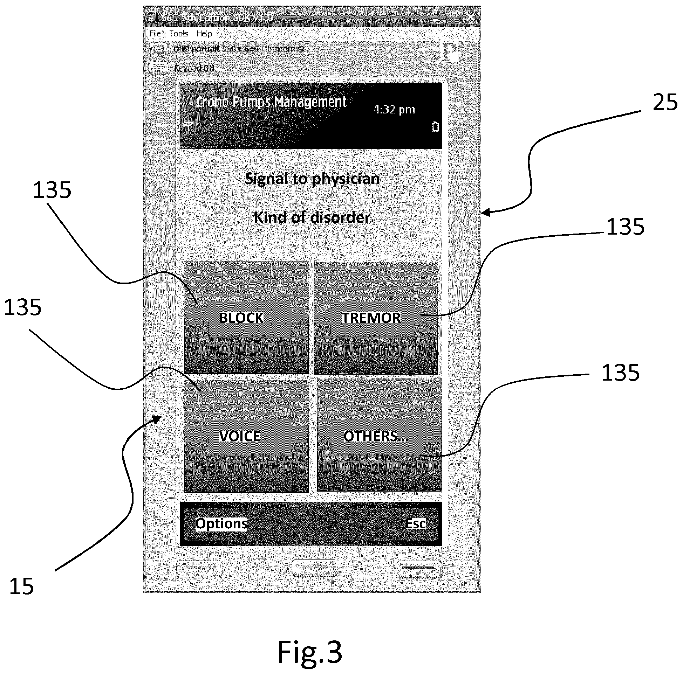

Referring to Fig.3 there is illustrated a second screen of a signaling and control interface associated with the signaling and control unit 15 equipped with touch-screen 25. Said interface provides for the display of a plurality of screen portions 135, associated with corresponding parameters, four parameters in the illustrated example, and corresponding to *"motor block", "tremor", "vocal disorder"* and *"others".* Each portion 135 delimits a corresponding area of the touch-screen 25. The interaction of the user's fingers with said portions 135 causes the sending of a data associated with the parameter displayed in the corresponding portion.

Said sending of data can be carried out immediately through the communication module 23 or 24 equipping the unit 15 or the data can be initially stored in a memory device inside unit 15 and subsequently forwarded through the modules 23 or 24. Data sent through modules 23 and 24 are received by unit 17, which in turn is equipped with a corresponding communication module 29 and 33 and employed therein to display graphics, as it will become clear from the following description.

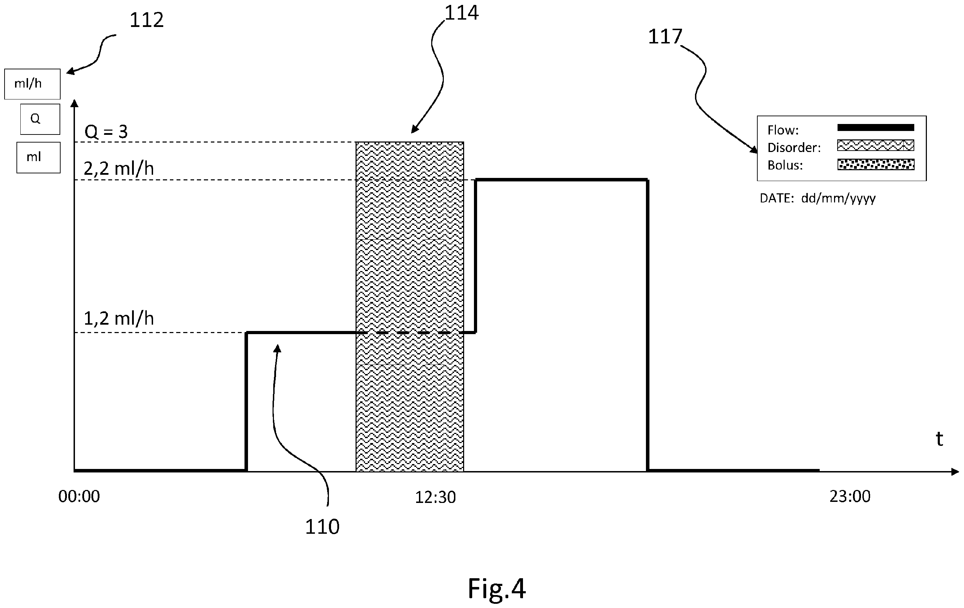

Referring to Fig.4 and, still by way of a non limiting example, with reference to a drug infusion unit, the method for controlling and programming an electromedical apparatus according to the invention will be disclosed.

According to the invention the method for controlling and programming a drug infusion unit, e.g. of the type identified by reference numeral 13 in Fig.1, comprises the steps of:
- displaying a first graph 110 indicative of the temporal variation according to a predetermined time scale (e.g. minutes or hours) of at least one operative parameter 112 of the electromedical apparatus 13, said parameter 112 being associated with the administration therapy with which the electromedical apparatus 13 is programmed to operate;
- displaying a second graph 114 indicative of a variation of the patient's conditions and/or of an operative parameter of the electromedical apparatus 13 and/or of a control command imparted through the signaling and control unit 15, wherein said first 112 and second 114 graphs are displayed overlapped, whereby the occurrence and the duration of the event associated with the second graph 114 are synchronized with the variation of the first parameter with respect to the time scale.

Therefore, according to the invention said first and second graphs have in common the time axis, are displayed on the same screen portion and have a transparent background so as to be appreciable simultaneously and be easily compared.

With reference in particular to the illustrated example, said first parameter 112 corresponds to the quantity of drug delivered per time unit (ml/h).

Moreover, still with reference to the illustrated example said second graph 114 is indicative of illness conditions or indisposition, e.g. associated with a tremor of the patient of level Q = 3.

According to a preferred embodiment of the invention, the data referred to the type of illness of the patient is sent to unit 15 and selected by the patient through the interface of Fig.3 and the data referred to the level Q associated with said status of illness, is sent by unit 15 and selected by interacting three consecutive times with the screen portion 135 associated with the corresponding status of illness.

According to the invention the different illness situations, e.g. *"motor block", "tremor", "vocal disorder"* and *"others"* can be displayed by means of specific graphic symbols, e.g. a different background for the areas corresponding to said illness conditions, or be associated with alphabetical labels indicative of the corresponding status, so as to allow a fast identification of the illness condition.

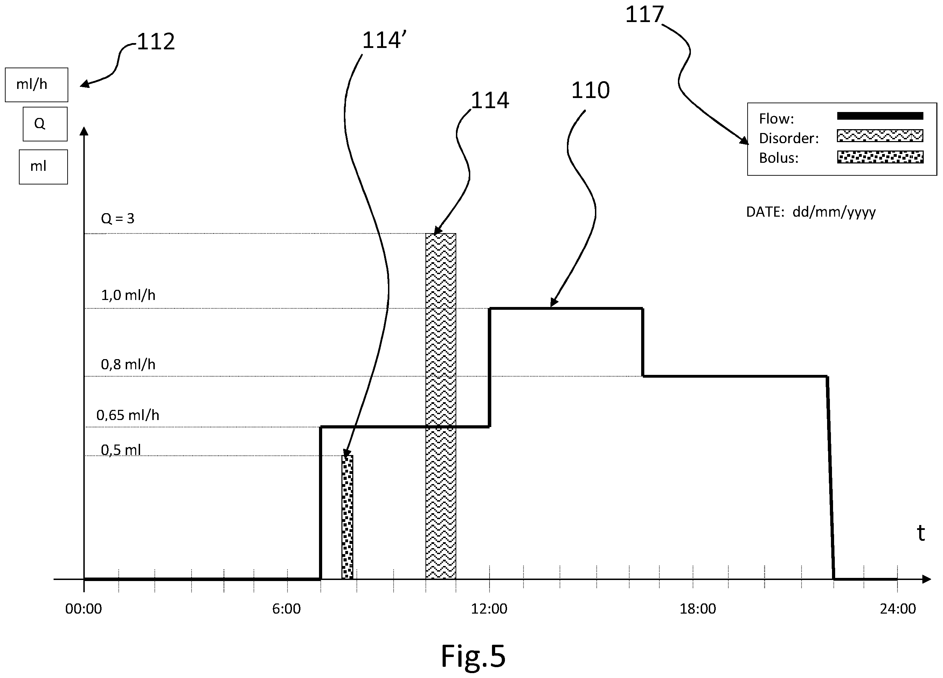

With reference to Fig.5, there is illustrated a situation similar to that of Fig.4 wherein a graph 114' indicative of the duration of a bolus administered by unit 13 to the patient following a command imparted by the patient through unit 15 is also present.

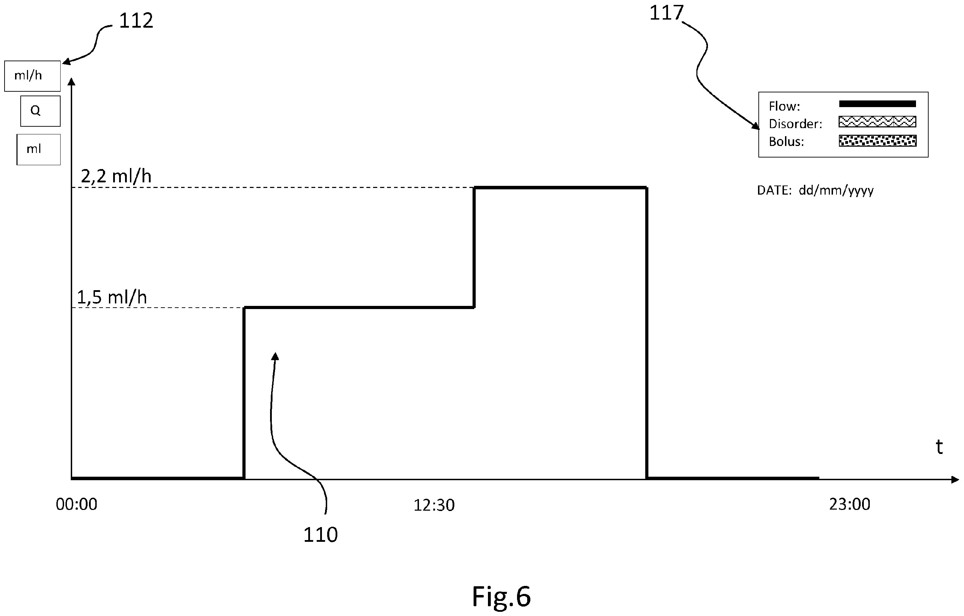

Still with reference to Fig.5 there is also illustrated a graph indicative of an illness condition and the graph 110 indicative of the variation of parameter 112, i.e. of the quantity of drug administered per time unit. According to the invention said graphs 110, 114, 114' are displayed with different modalities on the screen 31 so as to allow to distinguish said graphs one from the other. Moreover, as can be appreciated from Fig.5, the graphs 110, 114, 114' are overlapped, i.e. they have in common at least a part of the area subtended by the curve of the graph. To allow the information displayed on the same screen 31 to be interpreted a legend 117 is graphically provided on the screen 31. In a preferred embodiment of the invention, the method further provides for a step of programming the therapy. With reference to the illustrated example the programming step may comprise e.g. a modification of the parameter 112 at the time interval preceding the time at which the illness condition occurred. The illustrated example is based on the therapy of Fig.4 and the re-programmed therapy is shown in Fig.6 wherein it is illustrated that the drug flow is increased from 1,2 (therapy of Fig.4) to 1,5 ml/h (therapy of Fig.6) during the day, thereby causing the illness condition of the patient to disappear.

According to the invention, said programming step can be advantageously carried out rapidly in a graphical mode. More precisely, the programming step can be carried out by dragging vertically the horizontal line corresponding to the flow level to be modified, e.g. by dragging it with the cursor on the screen 31.

Therefore the method according to the invention will comprise a step of transmitting the new therapy to unit 13. Said transmission step can advantageously occur by means of communication modules and will comprise a first transmission step from remote unit 17 to local unit 15 and subsequently from local unit 15 to infusion unit 13.

Clearly, according to the invention, the programming of the new value of operative parameter 112, the drug hourly flow in the illustrated example, may consider the duration of the condition signaled by the patient, thereby causing the rapid adjustment of the therapy.

It is evident that the above description is solely given by way of non-limiting example and that variants and modifications can be made without departing from the scope of the invention, as defined in the appended claims.

### Translation of text matter in the figures:

### FIG. 2

SEGNALA AL MEDICO = SIGNAL TO PHYSICIAN
CONTROLLA INFUSORE = CHECK INFUSION DEVICE
Opzioni = Options
Esci = Esc

### FIG. 3

Segnala al medico = Signal to physician
Tipo di malessere = Kind of disorder
BLOCCO = BLOCK
TREMORE = TREMOR
VOCE = VOICE
ALTRO... = OTHERS...
Opzioni = Options
Esci = Esc

### FIGG. 4, 5, 6

Flusso = Flow
Malessere = Disorder
Bolo = Bolus
DATA= gg/mm/aaaa = DATE: dd/mm/yyyy

### FIG. 7

Ora = Time
Dyskinesia invalidante SI = Invalidating dyskinesia
Dyskinesia invalidante NO = Invalidating dyskinesia
Pompa ON = Pump ON
Pompa OFF = Pump OFF
(arte nota) = (prior art)

### FIG. 8

(arte nota) = (prior art)

## Claims

1. An architecture (11) for controlling and programming in real time a drug infusion pump (13) equipped with wireless communication module (21) for short-range communication comprising:
- a local signaling and control unit (15) equipped with wireless communication module (23) for short-range communication with said pump (13) and with wireless communication module (24) for long-range communication;
- a remote displaying and programming unit (17) comprising a personal computer (27) equipped with a screen (31), said unit being equipped with a wireless communication module (33) for long-range communication with said signaling and control unit (15), wherein said displaying and programming unit (17) is programmed for:
- displaying on said screen (31) of said displaying and programming unit (17) a first graph (110) indicative of the temporal variation according to a predetermined time scale of the quantity of drug (112) administered by the pump (13), said quantity (112) being associated with the administration therapy with which the pump (13) is programmed to operate;
- displaying on said displaying and programming unit (17) a second graph (114) indicative of a temporal variation of the patient's conditions and/or of a control command imparted through the signaling and control unit (15) for administering to the patient additional doses of drug (bolus), wherein said first (112) and second (114) graphs are displayed overlapped, i.e. they have in common at least a part of the area subtended by the curve of the graph, time axis, are displayed on the same screen portion and have a transparent background so as to be appreciable simultaneously and be easily compared, whereby the occurrence and the duration of the event associated with the second graph (114) are synchronized with the variation of the quantity of drug administered by the pump (13) with respect to the time scale;
- accepting control commands on said displaying and programming unit (17) for programming the quantity of drug to be administered per time unit by the pump, by means of a vertically draggable horizontal line with the cursor on the screen (31), said line corresponding to the flow level to be modified;
- transmitting the newly programmed therapy to infusion pump (13) by means of a first transmission step from remote unit (17) to local unit (15) and subsequently from local unit (15) to infusion pump (13).

2. An architecture according to claim 1, wherein the signaling and control unit (15) is associated with a handheld wireless communication mobile apparatus (19).

3. An architecture according to claim 1 or 2, wherein the signaling and control unit (15) comprises a touch-screen display (25), which allows the user to interact with the graphical interface by means of his fingers.

4. An architecture according to claim 3, wherein the signaling and control unit (15) is capable of transmitting signals indicative of the variation of the patient's conditions, said signals being imparted by interacting with said touch-screen display (25).

5. An architecture according to claim 3, wherein the signaling and control unit (15) is capable of receiving from the infusion pump (13) data associated with the current therapy, for displaying in a graphical form on said display (25) the current therapy, the remaining duration of the infusion or the level of the drug.

6. An architecture according to claim 3, wherein the signaling and control unit (15) is capable of controlling the infusion pump (13) for administering additional doses of drug.

7. A method for controlling and programming in real time a drug infusion pump equipped with wireless communication module (21) for short-range communication with a local signaling and control unit (15) equipped with wireless communication module (24) for long-range communication with a remote displaying and programming unit (17) comprising a personal computer (27) equipped with a screen (31) and equipped with a wireless communication module (33) for long-range communication, said method comprising the steps of:
- displaying on said screen (31) of said displaying and programming unit (17) a first graph (110) indicative of the quantity of drug administered per time unit by the pump (13), said quantity being associated with the administration therapy with which the pump (13) is programmed to operate;
- displaying on said displaying and programming unit (17) a second graph (114) indicative of a variation of the patient's conditions and/or of a control command imparted through the signaling and control unit (15) for administering to the patient additional doses of drug (bolus), wherein said first (112) and second (114) graphs are displayed overlapped i.e. they have in common at least a part of the area subtended by the curve of the graph, time axis, are displayed on the same screen portion and have a transparent background so as to be appreciable simultaneously and be easily compared, whereby the occurrence and the duration of the event associated with the second graph (114) are synchronized with the variation of the quantity of drug administered by the pump (13) with respect to the time scale;
- programming on said displaying and programming unit (17) the quantity of drug administered per time unit by the pump by means of a vertically draggable horizontal line with the cursor on the screen (31), said line corresponding to the flow level to be modified;
- transmitting the newly programmed therapy to infusion pump (13) by means of a first transmission step from remote unit (17) to local unit (15) and subsequently from local unit (15) to infusion pump (13).

8. A method according to claim 7, wherein said method comprises the step of displaying on a touch screen (25) provided on said signaling and control unit (15) a plurality of screen portions (135), associated with corresponding parameters corresponding to "motor block", "tremor", "vocal disorder" and "others", wherein each of said screen portions (135) delimit a corresponding area of the touch-screen (25), whereby the interaction of the user's fingers with said portions (135) causes the sending of a data associated with the parameter displayed in the corresponding portion through the long-range communication module (24) equipping the signaling and control unit (15) to displaying and programming unit (17), said data being employed therein to display said second (114) graph on said screen (31).

## Patentansprüche

1. Architektur (11) zum Steuern und Programmieren einer Medikamenteninfusionspumpe (13) in Echtzeit, die mit einem drahtlosen Kommunikationsmodul (21) zur Kommunikation über kurze Entfernungen versehen ist, mit:
- einer lokalen Sende- und Steuereinheit (15), die mit einem drahtlosen Kommunikationsmodul (23) zur Kommunikation mit der Pumpe (13) über kurze Entfernungen und mit einem drahtlosen Kommunikationsmodul (24) zur Kommunikation übergroße Entfernungen versehen ist,
einer entfernten Anzeige- und Programmiereinheit (17), die einen Kleincomputer (27) enthält, der mit einem Bildschirm (31) versehen ist, wobei die Einheit mit einem drahtlosen Kommunikationsmodul (33) zur Kommunikation über große Entfernungen mit der Sende- und Steuereinheit (15) versehen ist, wobei die Anzeige- und Programmiereinheit (17) programmiert ist zum:
- Anzeigen auf dem Bildschirm (31) der Anzeige- und Programmiereinheit (17) eines ersten Graphen (110), der eine zeitliche Änderung der von der Pumpe (13) verabreichten Menge des Medikaments (112) gemäß einer vorgegebenen Zeitskala anzeigt, wobei die Menge (112) der Verabreichungstherapie zugeordnet ist, nach welcher die Pumpe (13) programmgemäß arbeiten soll,
- Anzeigen auf der Anzeige- und Programmiereinheit (17) eines zweiten Graphen (114), der eine zeitliche Änderung des Zustands des Patienten und/oder einer Steueranweisung anzeigt, die von der Sende- und Steuereinheit (15) zum Verabreichen von zusätzlichen Medikamentendosen (Bolus) gegeben wurde, wobei die ersten (112) und zweiten (114) Graphen überlappend dargestellt werden, d.h. sie haben zumindest einen Teil der Fläche gemeinsam, die durch die Kurve des Grafen, Zeitachse, unterteilt ist, sind im selben Bildschirmabschnitt dargestellt und haben einen transparenten Hintergrund, sodass sie gemeinsam betrachtet werden und leicht verglichen werden können, wobei das Auftreten und die Dauer des Ereignisses, das dem zweiten Grafen (114) zugeordnet ist, mit der Änderung der Menge des durch die Pumpe (13) verabreichten Medikaments in Bezug auf die Zeitskala synchronisiert ist,
- Akzeptieren von Steuerbefehlen an die Anzeige- und Programmiereinheit (17) zum Programmieren der Menge des Medikaments, das pro Zeiteinheit von der Pumpe verabreicht werden soll, mittels einer senkrechten Linie, die mit dem Positionsanzeiger auf dem Bildschirm (31) verschoben werden kann, wobei die Linie dem zu ändernden Strömungswert entspricht,
- Übertragen des neu programmierten Therapieplans an die Infusionspumpe (13) mittels eines erstens Übertragungsschritts von der entfernten Einheit (17) zur lokalen Einheit (15) und anschließend von der lokalen Einheit (15) zur Infusionspumpe (13).

2. Architektur nach Anspruch 1, wobei die Anzeige- und Steuereinheit (15) einem mobilen Handgerät (19) zur drahtlosen Kommunikation zugeordnet ist.

3. Architektur nach Anspruch 1 oder 2, wobei die Anzeige- und Steuereinheit (15) einen Sensorbildschirm (25) aufweist, der es einem Benutzer ermöglicht, mit seinen Fingern auf eine graphische Schnittstelle einzuwirken.

4. Architektur nach Anspruch 3, wobei die Anzeige- und Steuereinheit (15) dazu in der Lage ist, Signale zu übertragen, die die Änderungen des Zustands des Patienten anzeigen, wobei die Signale durch Einwirken auf den Sensorbildschirm (25) übermittelt werden.

5. Architektur nach Anspruch 3, wobei die Anzeige- und Steuereinheit (15) dazu geeignet ist, von der Infusionspumpe (13) Daten zu erhalten, die mit dem aktuellen Therapieplan im Zusammenhang stehen, um auf der Anzeige (25) in graphischer Form, den aktuellen Therapieplan, die verbleibende Dauer der Infusion oder der Stufe des Medikaments anzuzeigen.

6. Architektur nach Anspruch 3, wobei die Anzeige- und Steuereinheit (15) dazu in der Lage ist, die Infusionspumpe (13) zum Verabreichen von zusätzlichen Dosen des Medikaments zu steuern.

7. Verfahren zum Steuern und Programmieren einer Medikamenteninfusionspumpe in Echtzeit, die mit einem drahtlosen Kommunikationsmodul (21) zur Kommunikation über kurze Entfernungen mit einer lokalen Sende- und Steuereinheit (15) versehen ist, die mit einem drahtlosen Kommunikationsmodul (24) zur Kommunikation über große Distanzen mit einer entfernten Anzeige- und Programmiereinheit (17) versehen ist, die einen Kleincomputer (27) aufweist, der mit einem Bildschirm (31) und einem drahtlosen Kommunikationsmodul (33) zur Kommunikation über große Entfernungen versehen ist, wobei das Verfahren die folgenden Schritte enthält:
- Anzeigen auf dem Bildschirm (31) der Anzeige- und Programmiereinheit (17) eines ersten Graphen (110), der die Menge des pro Zeiteinheit von der Pumpe (13) verabreichten Medikaments anzeigt, wobei die Menge mit der Verabreichungstherapie verknüpft ist, nach der die Pumpe (13) programmgemäß arbeiten soll,
- Anzeigen eines zweiten Graphen (114) auf der Anzeige- und Programmiereinheit (17), der eine Änderung des Zustandes des Patienten und/oder eines durch die Sende- und Steuereinheit (15) gegebenen Steuerbefehls zum Verabreichen von zusätzlichen Dosen (Bolus) des Medikaments an den Patienten, wobei die ersten (112) und zweiten (114) Graphen überlappend dargestellt werden, d.h. zumindest einen Teil der Fläche gemein haben, die durch den Verlauf des Graphen, der Zeitachse, unterteilt ist, auf demselben Bildschirmteil dargestellt werden und einen transparenten Hintergrund haben, sodass sie gemeinsam betrachtet und einfach verglichen werden können, wobei das Auftreten und die Dauer des mit dem zweiten Graphen (114) verbundenen Ereignisses mit der Änderung der Menge des von der Pumpe (13) verabreichten Medikaments in Bezug auf die Zeitskale synchronisiert ist,
- Programmieren auf der Anzeige- und Programmiereinheit (17) der Menge des Medikaments, das pro Zeiteinheit von der Pumpe verabreicht werden soll, mittels einer vertikalen Linie, die mittels des Cursors auf dem Bildschirm (31) vertikal verschoben werden kann, wobei die Linie den zu ändernden Strömungswert entspricht,
- Übertragen des neu programmierten Therapieplans an die Infusionspumpe (13) mittels eines ersten Übertragungsschrittes von der entfernten Einheit (17) zur lokalen Einheit (15) und anschließend von der lokalen Einheit (15) zur Infusionspumpe (13).

8. Verfahren nach Anspruch 7, wobei das Verfahren den Schritt des Darstellens auf einem Sensorbildschirm (25), der an der Sende- und Steuereinheit (15) vorgesehen ist, von mehreren Bildschirmbereichen (135), die entsprechenden Parametern zugeordnet sind, die einem "Motorblock", "Zittern", "Stimmenstörung" und "anderem" entsprechen, enthält, wobei jeder der Bildschirmbereiche (135) ein entsprechendes Gebiet auf dem Sensorbildschirm (25) begrenzt, wodurch das Einwirken der Finger eines Benutzers auf die Abschnitte (135) das Aussenden von Daten bewirkt, die dem in dem entsprechenden Abschnitt dargestellten Parameter zugeordnet sind, über das Kommunikationsmodul (24) großer Reichweite, das zu der Sende- und Steuereinheit (15) gehört, an die Anzeige- und Programmiereinheit (17), wobei die dabei verwendeten Daten verwendet werden, um den zweiten Graphen (114) auf dem Bildschirm (31) anzuzeigen.

## Revendications

1. Architecture (11) de commande et de programmation en temps réel d'une pompe d'infusion de médicament (13) équipée d'un module de communication sans fil (21) pour une communication à courte portée, comprenant :
- une unité de commande et de signalisation locale (15) équipée d'un module de communication sans fil (23) pour une communication à courte portée avec ladite pompe (13) et d'un module de communication sans fil (24) pour une communication à longue portée ;
- une unité de programmation et d'affichage à distance (17) comprenant un ordinateur personnel (27) équipé d'un écran (31), ladite unité étant équipée d'un module de communication sans fil (33) pour une communication à longue portée avec ladite unité de commande et de signalisation locale (15), dans laquelle ladite unité de programmation et d'affichage (17) est programmée pour :
- afficher sur ledit écran (31) de ladite unité de programmation et d'affichage (17) un premier graphique (110) indicatif de la variation temporelle selon une échelle de temps prédéterminée de la quantité de médicament (112) administrée par la pompe (13), ladite quantité (112) étant associée à la thérapie d'administration avec laquelle la pompe (13) est programmée pour fonctionner ;
- afficher sur ladite unité de programmation et d'affichage (17) un second graphique (114) indicatif d'une variation temporelle des états du patient et/ou d'un ordre de commande communiqué par le biais de l'unité de commande et de signalisation (15) pour administrer au patient des doses supplémentaires de médicament (bolus), dans laquelle lesdits premier (112) et second (114) graphiques sont affichés chevauchés, c'est-à-dire ils ont en commun au moins une partie de la zone sous-tendue par la courbe du graphique, l'axe de temps, sont affichés sur la même portion d'écran et ont un arrière-plan transparent de façon à être décelables simultanément et être comparés facilement, moyennant quoi la survenue et la durée de l'évènement associé au second graphique (114) sont synchronisées avec la variation de la quantité de médicament administrée par la pompe (13) par rapport à l'échelle de temps ;
- accepter des ordres de commande sur ladite unité de programmation et d'affichage (17) pour programmer la quantité de médicament à administrer par unité de temps par la pompe, au moyen d'une ligne horizontale entraînable à la verticale avec le curseur sur l'écran (31), ladite ligne correspondant au niveau de l'écoulement à modifier ;
- transmettre la thérapie nouvellement programmée à la pompe d'infusion (13) au moyen d'une première étape de transmission de l'unité à distance (17) à l'unité locale (15) et ensuite de l'unité locale (15) à la pompe d'infusion (13).

2. Architecture selon la revendication 1, dans laquelle l'unité de commande et de signalisation (15) est associée à un appareil mobile de communication sans fil portable (19).

3. Architecture selon la revendication 1 ou 2, dans laquelle l'unité de commande et de signalisation (15) comprend un afficheur à écran tactile (25), qui permet à l'utilisateur d'interagir avec l'interface graphique au moyen des doigts.

4. Architecture selon la revendication 3, dans laquelle l'unité de commande et de signalisation (15) est capable de transmettre des signaux indicatifs de la variation des états du patient, lesdits signaux étant communiqués par interaction avec ledit afficheur à écran tactile (25).

5. Architecture selon la revendication 3, dans laquelle l'unité de commande et de signalisation (15) est capable de recevoir en provenance de la pompe d'infusion (13) des données associées à la thérapie en cours, pour afficher sous forme graphique sur ledit afficheur (25) la thérapie en cours, la durée restante de l'infusion ou le niveau du médicament.

6. Architecture selon la revendication 3, dans laquelle l'unité de commande et de signalisation (15) est capable de commander la pompe d'infusion (13) pour administrer des doses supplémentaires de médicament.

7. Procédé de commande et de programmation en temps réel d'une pompe d'infusion de médicament équipée d'un module de communication sans fil (21) pour une communication à courte portée avec une unité de commande et de signalisation locale (15) équipée d'un module de communication sans fil (24) pour une communication à longue portée avec une unité de programmation et d'affichage à distance (17) comprenant un ordinateur personnel (27) équipé d'un écran (31) et équipé d'un module de communication sans fil (33) pour une communication à longue portée, ledit procédé comprenant les étapes de :
- affichage sur ledit écran (31) de ladite unité de programmation et d'affichage (17) d'un premier graphique (110) indicatif de la quantité de médicament (112) administrée par unité de temps par la pompe (13), ladite quantité (112) étant associée à la thérapie d'administration avec laquelle la pompe (13) est programmée pour fonctionner ;
- affichage sur ladite unité de programmation et d'affichage (17) d'un second graphique (114) indicatif d'une variation des états du patient et/ou d'un ordre de commande communiqué par le biais de l'unité de commande et de signalisation (15) pour administrer au patient des doses supplémentaires de médicament (bolus), dans lequel lesdits premier (112) et second (114) graphiques sont affichés chevauchés, c'est-à-dire ils ont en commun au moins une partie de la zone sous-tendue par la courbe du graphique, l'axe de temps, sont affichés sur la même portion d'écran et ont un arrière-plan transparent de façon à être décelables simultanément et être comparés facilement, moyennant quoi la survenue et la durée de l'évènement associé au second graphique (114) sont synchronisées avec la variation de la quantité de médicament administrée par la pompe (13) par rapport à l'échelle de temps ;
- programmation sur ladite unité de programmation et d'affichage (17) de la quantité de médicament administrée par unité de temps par la pompe au moyen d'une ligne horizontale entraînable à la verticale avec le curseur sur l'écran (31), ladite ligne correspondant au niveau d'écoulement à modifier ;
- transmission de la thérapie nouvellement programmée à la pompe d'infusion (13) au moyen d'une première étape de transmission de l'unité à distance (17) à l'unité locale (15) et ensuite de l'unité locale (15) à la pompe d'infusion (13).

8. Procédé selon la revendication 7, dans lequel ledit procédé comprend l'étape d'affichage sur un écran tactile (25) prévu sur ladite unité de commande et de signalisation (15) d'une pluralité de portions d'écran (135), associées à des paramètres correspondants qui correspondent à « blocage moteur », « tremblement », « trouble vocal » et « autres », dans lequel chacune desdites portions d'écran (135) délimite une zone correspondante de l'écran tactile (25), moyennant quoi l'interaction des doigts de l'utilisateur avec lesdites portions d'écran (135) provoque l'envoi d'une donnée associée au paramètre affiché dans la portion correspondante par le biais du module de communication à longue portée (24) équipant l'unité de commande et de signalisation (15) à l'unité de programmation et d'affichage (17), ladite donnée étant employée ici pour afficher ledit second graphique (114) sur ledit écran (31).
